# EUROPEAN PATENT SPECIFICATION

(11) **EP 1 917 923 B1**
(45) Date of publication and mention of the grant of the patent: **20.11.2013**
(21) Application number: 06775496.0
(22) Date of filing: 25.08.2006
(51) Int. Cl.: A61B 17/326

(54) **AN APPARATUS FOR CIRCUMCISING A PENIS**
GERÄT ZUM BESCHNEIDEN EINES PENIS
APPAREIL DE CIRCONCISION DU PÉNIS

(30) Priority: 26.08.2005 CN 200520106272 U
(43) Date of publication of application: 07.05.2008
(73) Proprietor: Shang, Jianzhong, Xinwu District, Wuhu, Anhui 241000 (CN)
(72) Inventor: Shang, Jianzhong, Xinwu District, Wuhu, Anhui 241000 (CN)
(74) Representative: Cooke, Tracey
(86) International application number: PCT/CN2006/002178
(87) International publication number: WO 2007/022730

(56) References cited:
- WO-A1-2005/039424
- CN-Y- 2 249 064
- CN-Y- 2 662 842
- CN-Y- 2 694 913
- CN-Y- 2 815 285
- CN-Y- 2 815 286
- US-A- 2 353 647
- US-A- 3 435 823
- US-A- 5 269 788
- US-A- 5 797 921

## Description

### Field of the Invention

The invention relates to a type of medical apparatus, especially the provision of a type of circumcision apparatus.

### Background and Prior Art

Redundant prepuce or phimosis is one of the origins of male urinary system infection and sexually transmitted diseases. Redundant prepuce or phimosis can cause urinary tract infection that will lead to chronic prostatitis, which will incur a series of symptoms like back pain, impotence and prospermia, etc. Therefore, removing redundant foreskin or phimosis is one of the good measures to prevent these diseases.

Regarding the removal of redundant foreskin or phimosis, there have been several reforms from the early days of the founding of PRC to the present, but the operation will inevitably lead to bleeding, pain and hospitalization. Circumcision is one of the traditional measures to cure phimosis and redundant prepuce and prevent their complication, which belongs to the basic operation of male urological department and its technical essential is the three major steps of redundant foreskin removal, hemostasia, and apposition suture at the incisal edge. After the operation, the patient can not walk, and he will feel intolerably painful at each change of dressing and suffer great pain in the end at removal of stitches. Furthermore, if hemostasis by ligation is done by halves, it will cause preputial hematoma, then another surgical treatment is necessary. Though laser and high-frequency electric-knife technology have been used in foreskin circumcision, they have only substituted scissors cutting and solidified the bleeding spot. Such a therapeutic method is likely to subject the patient to tissular bum and infection, while the expenditure for infection prevention is very high. Judged from the comparison among various provinces and cities, whenever laser circumcision is performed, treatment by antibiotics after the operation for 5-7days plus prevention of infection by taking antibiotics orally for 5-7 days is very common, while the cost generally ranges from150 to 360 yuan, and the total cost of treatment is at least 1000-1500 yuan.

The existing technology has produced some solutions of circumcision apparatus. These circumcision apparatus are simple and easily applicable, and no surgery and suture is necessary. As a result, the pain is very light, the patient's work and life will not be affected, and the operation time is short. Nevertheless, these circumcision apparatus have the following common defects:
1. A type of circumcision apparatus, its blade conjoint part of the two semi-ferrules of the retaining ring is of right-angle structure. When the penis is clamped with two semi-ferrules, the foreskin will be easily clamped by the conjoined part of the blade, which makes the patient very painful.
2. Since it is inevitable for infection to occur during circumcision, medicament is needed to cooperate with the treatment so as to eliminate inflammation and grow the skin, etc. However, the existing circumcision apparatus itself does not design the corresponding structure, and the anti-inflammation treatment is carried out by such traditional external antiphlogistic manner as injection, oral administration, etc.
3. The locking device of the existing circumcision apparatus generally adopts screw fixation , therefore manual manipulation is very inconvenient, and it is extremely afflictive for the patients who will undergo the surgical operation, which increases their fear of operation and lengthens the operation time. Several kinds of connecting structure are introduced as follows.
4. The balanus ferrule of existing circumcision apparatus is a circular structure, however, the penis will inevitably erect and enlarge in the entire course of treatment and such a structure with fixed balanus ferrule diameter adds to the discomfort of the patients.
5. A type of circumcision apparatus, which includes a retaining ring and auxiliary ring. Put the glans penis through the auxiliary ring, and place the foreskin between the auxiliary ring and the retaining ring, and then tie it to the auxiliary ring with the binding string. In this way, when the penis is expanding, because the elasticity of the binding string is weak, and its contact area with the foreskin is small, the binding string and the foreskin may move relatively, resulting in incomplete blockage of the blood stream, which will produce hematoma and affect the surgical effect.
6. Also, as stated in item 5 , during real operation, since the binding string is bound by hand, it is often operated entirely by right of experience according to the habit of the operator. As a result, the degrees of tightness are varied, resulting in non-standardization of the entire process of operation, which in turn affects the effect of the surgery.

WO 2005/039424 Al discloses an apparatus for circumcising a penis, including a clamping mechanism, a rubber ring, and a glans ring or balanus ferrule, wherein the rubber ring is placed between the glans ring and the clamping mechanism.

US 3 435 823 A discloses an anastomotic coupling for connecting two bodily vessels.

US 2 353 647 A discloses an apparatus for clamping the foreskin during circumcision.

### Summary of the Invention

The main purpose of this invention lies in overcoming the deficiency of the existing technology and providing a kind of novel disposable apparatus for circumcising a penis that requires no surgery during treatment period with no bleeding, no suture, no medication, no pain, low cost and handy and comfortable use.

In order to realize the abovementioned purpose, this invention adopts a technical solution as follows: a type of circumcision apparatus, which includes a fastening device and a balanus ferrule, of which: the fixture has an opening, and mutually corresponding upper-blade conjoint part and lower-blade conjoint part are provided on either end of the opening. Round angles are provided at the edges of the upper blade conjoined part and the lower blade conjoined part.

Two or more the first grooves are provided at the edges of the balanus ferrule and the first protuberance is formed among the first grooves;

There are two or more the second protuberances that interact with the first grooves at the inner edge of the fixture device and the second grooves are formed among the second protuberances;

The second grooves and the first protuberances interact to form a cavity to hold medicine solution; and a passage for injection is formed on the fixture device and interconnects with the cavity.

The balanus ferrule is composed of right and left semi-ferrules that are separated from each other; a round angle is provided at the connexion of right and left semi-ferrules.

The first scalariform detent block is installed at tip of the opening with Upper blade conjoined part, while the second scalariform detent block is installed at the tip of the opening with Lower blade conjoined part; and the first scalariform detent block interacts with the second scalariform detent block.

The first scalariform detent block lies under Upper blade conjoined part, while the second scalariform detent block lies above Lower blade conjoined part.

The fixture device is composed of two semi-ferrules and a Connecting buckle. These two semi-ferrules are inter-connected at one end through the Connecting buckle, while the other ends of the two semi-ferrules are locked with a screw rod. One semi-ferrule of the two has an upper blade conjoined part. The other semi-ferrule has a lower blade conjoined part that corresponds with the upper blade conjoined part. Round angles are provided at the edges of the upper blade conjoined part and the lower blade conjoined part.

The fixture device is composed of two semi-ferrules and a Connecting buckle. These two semi-ferrules is inter-connected at one end through the Connecting buckle, while the other ends of the two semi-ferrules are provided with L-shaped parts interacting with each other. Connecting rod shaft is installed at the top of L-shaped parts. Attachment holes are provided at the bottom platform of the L-shaped parts. The Connecting rod shaft interacts with the attachment hole of the other semi-ferrule. Toothlike protuberance or toothlike groove is provided at the inner side of the upstanding end of L-shaped part. The toothlike protuberance or toothlike groove interacts with that of the other semi-ferrule. One semi-ferrule of the two has an upper blade conjoined part. The other semi-ferrule has a lower blade conjoined part that corresponds with the upper blade conjoined part. Round angles are provided at the edges of the upper blade conjoined part and the lower blade conjoined part. One or more connecting rod shaft is installed at the top of L-shaped parts, flexible connection or permanent connection.

The fixture device is composed of two semi-ferrules and a Connecting buckle. These two semi-ferrules is inter-connected at one end through the Connecting buckle, while the other ends of the two semi-ferrules are provided with uncinate heave or slotted hole respectively. The uncinate heave or slotted hole interacts with that of the other semi-ferrule. The uncinate heave consists of joint plate and Clamping piece which is of Λ-shaped structure. The joint plate connects with the tip of semi-ferrule at one end and with the central part of Clamping piece at the other end. One semi-ferrule of the two has an upper blade conjoined part. The other semi-ferrule has a lower blade conjoined part that corresponds with the upper blade conjoined part. Round angles are provided at the edges of the upper blade conjoined part and the lower blade conjoined part.

The fixture device is composed of two semi-ferrules and a Connecting buckle. These two semi-ferrules are inter-connected at one end through the Connecting buckle, while the other ends of the two semi-ferrules are provided with saucer shape heave respectively. The saucer shape heave contains a block in the form of a bow tie. One semi-ferrule of the two has an upper blade conjoined part. The other semi-ferrule has a lower blade conjoined part that corresponds with the upper blade conjoined part. Round angles are provided at the edges of the upper blade conjoined part and the lower blade conjoined part.

The fixture device is composed of two semi-ferrules and a Connecting buckle. These two semi-ferrules is inter-connected at one end through the Connecting buckle, while the other ends of the two semi-ferrules are provided with strip-shaped gear body or strip-shaped sprocket hole respectively. The strip-shaped gear body or strip-shaped sprocket hole interacts with that of the other semi-ferrule. One semi-ferrule of the two has an upper blade conjoined part. The other semi-ferrule has a lower blade conjoined part that corresponds with the upper blade conjoined part. Round angles are provided at the edges of the upper blade conjoined part and the lower blade conjoined part.

The highly efficient and hygienic germfree material enables the patient to keep from infection when in use which will bring about greater pain and unnecessary trouble. Also, this invention is rendered as disposables which are more hygienic and safer..

The apparatus for circumcising a redundancy provided by this invention retains the characteristics of no surgical operation, no suture and low degree of pain that are intrinsic of the circumcision apparatus, at the same time a great deal of improvement has been made addressing the many deficiencies of the existing circumcision apparatus, which, to be specific, includes:
1. An existing circumcision apparatus, its blade conjoint part of the two semi-ferrules of the retaining ring is of right-angle structure. When the penis is clamped with two semi-ferrules, the foreskin will be easily clamped by the conjoined part of the blade, which makes the patient very painful. The round angle structure at the blade conjoint part and ferrule combinative structure of this invention will avoid the foreskin being clamped during the operation and pain incurred to the patient, making the operation smoother. In addition, the upper and lower structure at the blade-conjoined part can better facilitate the clamping.
2. The balanus ferrule of the existing circumcision apparatus is an integrative circular structure, however, the penis will inevitably erect and enlarge in the entire process of treatment, as a result, such a structure with fixed balanus ferrule diameter adds to the discomfort of the patients. This invention transforms the single integral structure of the balanus ferrule into 2 sections (even more sections) of left and right semi-ferrules, thereby enabling adaptation to erection and enlargement of the penis.
3. The locking device of the existing circumcision apparatus generally adopts screw fixation, therefore manual manipulation is very inconvenient. This invention, through its flexible and handy 'push-buckle' structure, renders the operation more flexible and convenient, increases the comfort of the patient when in use, and is more humanized.
4. The existing circumcision apparatus itself does not design the corresponding structure, and the anti-inflammation treatment is carried out by such traditional external antiphlogistic manners as injection, oral administration, and so on. The invention enables the device to be more practical through provision of cavity and injection passage.
5. The existing circumcision apparatus includes a retaining ring and auxiliary ring, Put the glans penis through the auxiliary ring, and place the foreskin between the auxiliary ring and the retaining ring, and then tie it to the auxiliary ring with the binding string. In this way, because the elasticity of the binding string is weak, and its contact area with the foreskin is small, when the penis is expanding, the binding string and the foreskin may move relatively, resulting in incomplete blockage of the blood stream, which will produce hematoma and affect the surgical effect. The latex pad of this invention, because of its relatively strong elasticity, can mitigate in part the binding force when the penis expands. Also, since the contact area between the latex pad and the foreskin is rather large, no relative move between the latex pad and the blade fixture will occur when the penis is enlarging, yielding good surgical result.
6. Also, as stated in item 5, during real operation, since the binding string is bound by hand, it is often operated entirely by right of experience according to the habit of the operator. As a result, the degrees of tightness are varied, resulting in non-standardization of the entire process of operation, which in turn affects the effect of the surgery. The invention utilizes the latex pad, enabling standardization in the entire process, avoiding the problem caused by binding operation using the binding string manually.

This invention is small and exquisite in shape, light in weight, simple in structure, convenient in use, and low in price, with a total expense of only 160-200 yuan. Moreover, it is painless, has no ill scar after the circular cast of the foreskin, and the circular-casting position is smoother, cleaner and more accurate, well received by the great majority of sufferers.

Further description of the invention is made below in combination with the attached figures and concrete implementation manners.

### Brief Description of the Drawings

Fig 1A illustrate the fixture device of the apparatus for circumcising a penis;
Fig 1B illustrate the balanus ferrule of the apparatus for circumcising a penis ;
Fig 2 illustrate the section I-I of the fixture device of the apparatus for circumcising a penis ;
Fig 3 illustrate the section II-II of the balanus ferrule of the apparatus for circumcising a penis;
Fig 4A is the enlarged schematic diagram which illustrate Part A of the fixture device of the apparatus for circumcising a penis ;
Fig 4B is another enlarged schematic diagram which illustrate Part A of the fixture device of the apparatus for circumcising a penis ;
Fig 4C is another enlarged schematic diagram which illustrate Part A of the fixture device of the apparatus for circumcising a penis;
Fig 4D is again another enlarged schematic diagram which illustrate Part A of the fixture device of the apparatus for circumcising a penis ;
Fig 5 is the breakdown schematic diagram for another implementation example of the apparatus for circumcising a penis.
Fig 6 is the structural representation of the fixture device of the apparatus for circumcising a penis, another implementation example of this invention;
Fig 7 is structural representation for the conjoint part of the upper and lower blades of the circumcision apparatus III - III cross-section, another implementation example of this invention.
Fig 8 is the structural diagrammatic sketch for the fixture device of the apparatus for circumcising a penis, another implementation example of this invention; and
Fig 9 is the schematic diagram of the enlarged crosssectional structure of Part A in Fig 8.

### Detailed Description of the Preferred Embodiments of the Invention

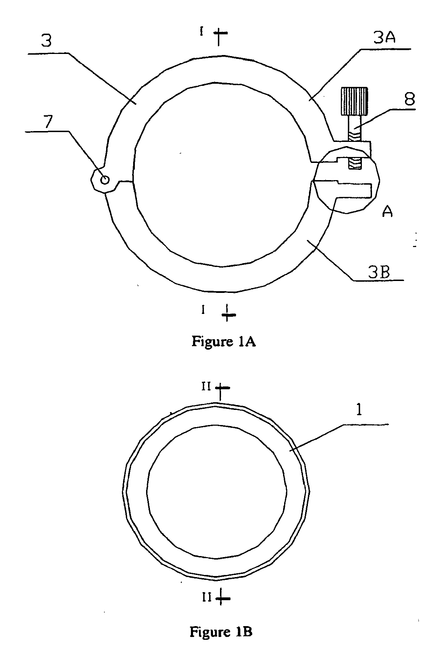

The implementation example of this invention is the disposable apparatus for circumcising a penis, as shown in Fig 1A and Fig 1B. The circumcision apparatus includes the balanus ferrule 1 (not divided into sections temporarily), its exterior being equipped with the fixture device 3, and the raw material of the balanus ferrule1 and fixture device 3 is the high-grade 372-type organic material without toxic and side effect.

The fixture device 3 is comprised of the left and right semi-ferrules 3A and 3B, and connecting buckles 7 are installed at both ends of the semi-ferrules , with the other end possessing the connecting and fixing parts. Of course, the fixture device 3 can also be an integral structure, with openings, which is connected through the fixing part. The fixing part is the fix screw 8 or other connecting forms, which will be exemplified in the following introduction.

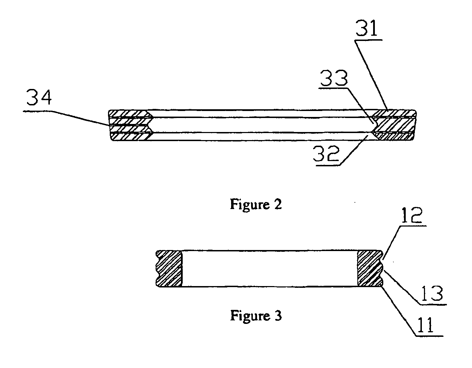

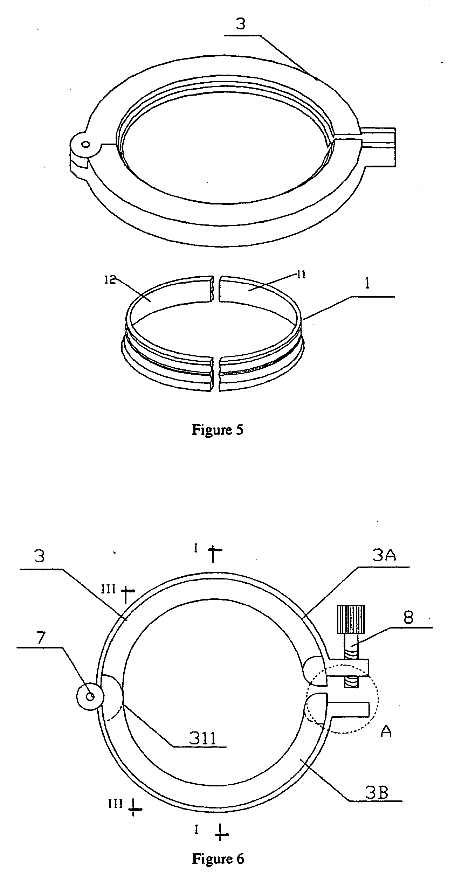

Fig 2 illustrate the section I-I of the fixture device of the apparatus for circumcising a penis ; Fig 3 illustrate the section II-II of the balanus ferrule of the apparatus for circumcising a penis; As shown in the figure, one of the key points of this invention lies in: the balanus ferrule of existing circumcision apparatus is a circular structure, and such a structure with fixed balanus ferrule diameter adds to the discomfort of the patients. Shown in Fig 5 is the breakdown schematic diagram for another implementation example of the apparatus for circumcising a penis, which transforms the single integral structure of the balanus ferrule into 2 sections of left and right half ferrules 11 and 12, thereby enabling adaptation to erection and enlargement of the penis, and the squeeze can also be alleviated.

Besides, the outer edge 11 of the balanus ferrule 1 is equipped with two or over two (two for this implementation example) the first grooves 12, between which the first protuberance 13 is formed; the inner edge 31 of the fixture device 3 has two or over two (two for this implementation example) second saliencies 32 that match with the first grooves 12 in question, and the second grooves 33 is formed between the second saliencies 32 , and the second groove 33 coordinates with the first protuberance 13 to form a cavity that is used to hold liquid drug; the passage 34 for injection of drug liquid is formed on the fixture device 3, which is connected with the cavity. The quantity of the passage 34 can be set as several according to the condition of use. During operation the aforementioned the first groove 12, the second groove 33, first protuberance 13 and the second protuberance 32 coordinate with each other to clamp the foreskin to be circumcised, resulting in its necrosis due to unsmooth blood circulation. Since it is inevitable for infection to occur during circumcision, medicament is needed to cooperate with the treatment so as to eliminate inflammation and grow the skin, etc. The drug liquid in the above cavity directly permeates into the infected part to eliminate the inflammation and grow the skin, enabling a shorter time for postoperative concrescence. The formula of these liquid drugs is specially prepared for circumcision, and will not be dealt with again here as it is not related to this patent application.

Since the fastening operation for the connecting part A of the existing fixture device 3 is very inconvenient, and it is extremely afflictive for the patients who will undergo the operation, which increases their fear of operation and lengthens the operation time. Several kinds of connecting structure are introduced as follows.

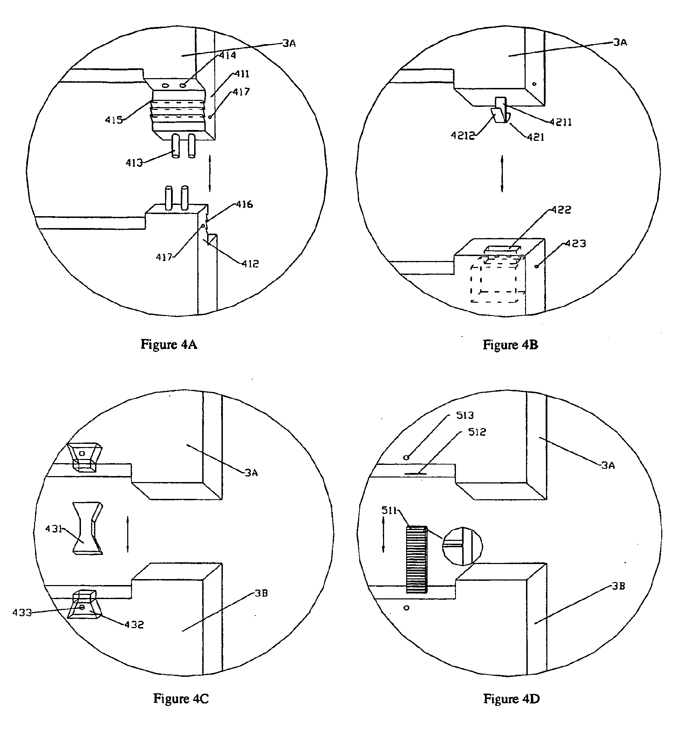

Fig 4A is the enlarged schematic diagram which illustrates Part A of the fixture device of the apparatus for circumcising a penis; The aforesaid fixture device 3 consists of the two semi-ferrules of 3A & 3B and the Connecting buckle 7. These two semi-ferrules of 3A & 3B are inter-connected at one end through connecting buckle 7, while the other ends of the two semi-ferrules are provided with coordinated L-shaped parts of 411 & 412 respectively. Connecting rod shaft 413 is installed at the top of L-shaped parts of 411 & 412. Attachment hole 414 is provided at the bottom platform. Connecting rod shaft 413 interacts with Attachment hole 414 of the other semi-ferrule. Toothlike protuberance 415 or toothlike groove 416 is provided at the inner side of the upstanding end of L-shaped part. The toothlike protuberance 415 or toothlike groove 416 interacts with that of the other semi-ferrule. Thus,when locking connection of the two semi-ferrules of 3A & 3B is needed, you only need to plant connecting rod shaft 413 inside attachment hole 414 and make toothlike protuberance 415 and toothlike groove 416 on the other L-shaped part joggle to each other at the same time in order to achieve the locking of the whole fixture device 3. Of course, you can insert a screwdriver or other tools inside Hole 417 on L-shaped part if connection opening is required. And with little efforts, you will break down the whole structure and open up the implementation example, which is a disposable circumcision apparatus.

Fig 4B is another enlarged schematic diagram which illustrates Part A of the fixture device of the apparatus for circumcising a penis; The aforesaid fixture device 3 consists of the two semi-ferrules of 3A & 3B and the connecting buckle 7. These two semi-ferrules of 3A & 3B are inter-connected at one end through connecting buckle 7, while the other ends of the two semi-ferrules are provided with uncinate heave 421 or slotted hole 422. uncinate heave 421 or slotted hole 422 interacts with that of the other semi-ferrule. Uncinate heave 421 consists of Joint plate 4211 and clamping piece 4212 which is of Λ-shaped structure. Joint plate 4211 connects with the tip of semi-ferrule 3A at one end and with the central part of clamping piece 4212 at the other end. Clamping piece 4212 is of elastic structure with certain degree of hardness, e. g. sheet bar. Slotted hole 422 boasts large jaw opening and large interior cavity. At the tip of Semi-ferrule 3A, there is Hole 423 which has the same function with Hole 417 in FIG 4A. When locking, insert uncinate heave 421 into Slotted eye 422. Here,elastic property of clamping piece 4212 works. After Clamping piece 4212 is fixed, its hardness allows it to lean against the interior cavity wall of Slotted hole 422, thus locking is achieved.

Fig 4C is another kind of structural enlargement schematic diagram for Part A of the fixture of the circumcision apparatus, the implementation example of this invention. The aforesaid fixture device 3 consists of the two semi-ferrules of 3A & 3B and the connecting buckle 7. These two semi-ferrules of 3A & 3B are inter-connected at one end through connecting buckle 7, while the other ends of the two semi-ferrules are provided with saucer shape heave 432. The saucer shape heave 432 contains block 431 in the form of a bow tie. At the tip of semi-ferrule 3A, there is hole 433 which has the same function with aforementioned Hole 417 & 423. When locking, you only need to insert both ends of bow-tie shaped block 431 into saucer shape heave 432.

Fig 4D is again another kind of structural enlargement schematic diagram for Part A of the fixture of the circumcision apparatus, the implementation example of this invention. The aforesaid fixture device 3 consists of the two semi-ferrules of 3A & 3B and the Connecting buckle 7. These two semi-ferrules of 3A & 3B are inter-connected at one end through Connecting buckle 7, while the other ends of the two semi-ferrules are provided with strip-shaped gear body 511 or strip-shaped sprocket hole 512. As shown in the figure for detail enhancement of strip-shaped gear body 511, there are many horizontal lines of heave to interact with the petty groove arranged horizontally in strip-shaped gear body 511. When Strip-shaped gear body 511 is inserted inside Strip-shaped sprocket hole 512, it happens that the petty horizontal lines of heave of the strip-shaped sprocket hole are clamped into the petty groove of the strip-shaped gear body. It is noteworthy that such inserting is unidirectional, thus it is necessary to keep an obtuse angle between the tilt direction of and the inserting direction of the petty horizontal lines of heave of the strip-shaped sprocket hole and the petty groove of the strip-shaped gear body. Once they are clamped and fixed, there is normally no way to simply draw Strip-shaped gear body 511 away from Strip-shaped sprocket hole 512. So the fastness of the clamping is guaranteed, thus enhancing the fastness, stability and operability of this present invention. As for its disassembly after the operation is completed, this implementation example still adopt the hole structure designed to break the connection as aforementioned. You can insert a screwdriver or other tools inside Hole 513 if connection breakdown is required. And with little efforts, you can break down the whole structure and open up the implementation example.

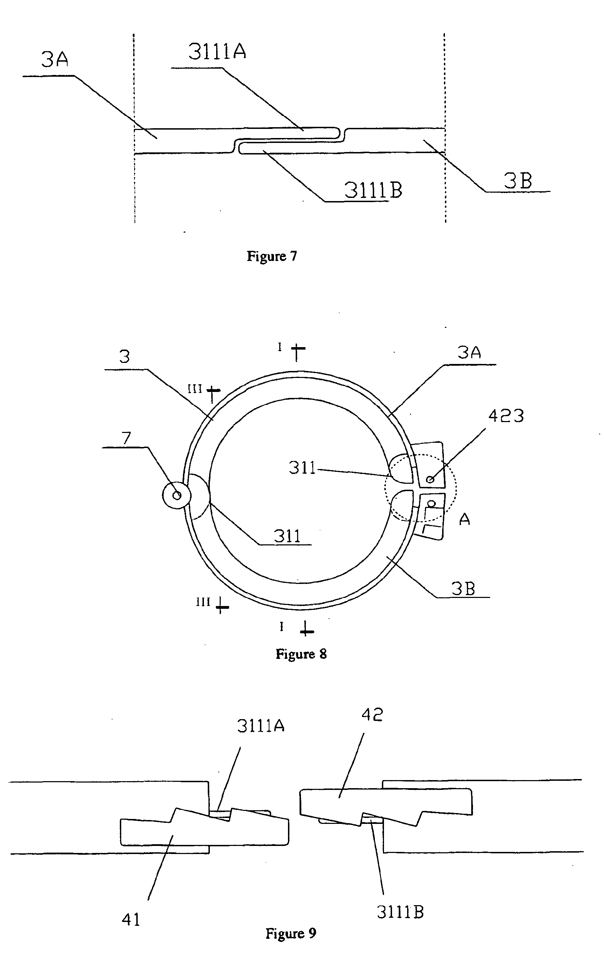

Fig 8 is the structural diagrammatic sketch for the fastening device of the circumcision apparatus, another implementation example of this invention. The aforementioned fixture device 3 has an opening. An upper blade conjoined part 3111A and a lower blade conjoined part 3111 B are provided on either end of the opening. The first scalariform detent block 41 is installed at tip of the opening with Upper blade conjoined part 3111 A, while the second scalariform detent block 42 is installed at the tip of the opening with Lower blade conjoined part 3111B. First scalariform detent block 41 and second scalariform detent block 42 interact with each other. In the profile direction,the first scalariform detent block 41 lies under Upper blade conjoined part 3111A, while the second scalariform detent block 42 lies above Lower blade conjoined part 3111B. When in use, insert Lower blade conjoined part 3111 B below Upper blade conjoined part 3111A as shown in FIG 7 & FIG 9. The detent of Second scalariform detent block 42 is achieved above the first scalariform detent block 41, resulting in the tight coupling of first scalariform detent block 41 with second scalariform detent block 42 by spacing of Upper blade conjoined part 3111A and Lower blade conjoined part 3111B. Thus, tight locking is achieved for the whole fixture device. There is Hole 423 on either first scalariform detent block or Second scalariform detent block. you can insert a screwdriver or other tools inside Hole 423 if connection opening is required. And with little efforts, you will break down the whole structure and open up the implementation example, which is a disposable apparatus for circumcising a penis.

With different linkage structures introduced, we now make the following explanations on the operation & use procedures of this present invention:
1. Sterilization treatment of the genitals; fully expose the foreskin by pushing back the foreskin that covers the balanus.
2. Adjust the position of the balanus ferrule, and pull out the back-pushed foreskin until it fully covers the balanus ferrule and the balanus.
3. Open the fixture and put it in from one side of the patient to let the fixture envelop and fix the balanus ferrule before the fixture is fixed, thus the foreskin is prevented from blood circulation and its necrosis is caused.
4. Injecting medicine solution into the cavity through Passage 34. The medicine solution can directly reach the affected part through the cavity, thereby diminishing inflammation and killing pain.
5. After 2-5 days(sometimes longer), take off the necrotic foreskin and loosen the fixture at the same time or later on.

In another implementation example, Semi-ferrule 3A of Fixture device 3 is provided with Upper blade conjoined part 3111A as shown in FIG 6 & 7. The other semi-ferrule 3B is provided with Lower blade conjoined part 3111B that corresponds with Upper blade conjoined part 3111A. The upper and lower blade conjoined parts can better facilitate the clamping of Fixture device 3. Round angle 311 is provided at the edges of the upper blade conjoined part and the lower blade conjoined part. The round angle structure will prevent the foreskin from being clamped during the operation and avoid the pain incurred to the patient. If the foreskin is clamped, it will become granulated. The recovery period will be longer and the patient will suffer more pain. In a similar way, the connexion of the two semi-ferrules on both right and left sides of Balanus ferrule 1 may be provided with the same or similar round angles in order to prevent the foreskin from being clamped.

This present invention is used without any pain. There will be no obvious scar left following the circular cast of the foreskin with quick recovery and accurate circular cast position. Children, youngsters and old men all can receive this treatment with different sizes of the apparatus applied according to the actual balanus sizes of patients.

In addition, patients' work or study is not affected during the whole process of the treatment. Being simple and needing no special training, the operation may be carried out in small clinics. The total period of treatment costs only 160 - 200 Yuan, including that of operation, antiinflammation, etc. Thus it is acceptable to the general public.

To sum up, the present invention is expounded from the aspects of its application target, effectivity, evolutionarity and novelty,etc. It has a practical evolutionarity that has already been in compliance with the requirements for function improvement and utility highlighted in laws on patented inventions. The above-mentioned implementation explanation and figures attached to the present invention are considered as one preferred implementation example of the invention, which should not be regarded as a limit to it. Therefore, all the structures, devices, features, etc that are same or similar to those of the present invention shall fall within the scope of the creation objective of the invention and its patent application.

## Claims

1. An apparatus for circumcising a penis, comprising:
a fixture device (3) and a balanus ferrule (1);
wherein said fixture device is closeable, and when not closed defines an opening;
**characterised in that** the fixture device is provided with conjoined upper and lower blade parts (3111A, 3111B), the upper and lower blade parts being arranged on either side of said opening,
and free ends of the upper and lower blade parts are rounded (311) to prevent the foreskin from being clamped during operation and overlap in use.

2. An apparatus as claimed in claim 1, **characterized in that** at least two first grooves (12) are provided at an edge of the balanus ferrule and a first protuberance (13) is formed between said first grooves;
at least two second protuberances (32) that interact with said first grooves are provided at an inner edge of the fixture device (1) and a second groove (33) is formed between the second protuberances;
the second grooves and the first protuberances interact to form a cavity to hold a medicine solution;
and a passage (34) for injection is formed on the fixture device and interconnects with the cavity.

3. An apparatus as claimed in claim 1 or 2, **characterized in that** the said balanus ferrule (1) is composed of right and left semi-ferrules (11, 12) that are separated from each other, and a round angle is provided at a connection of the right and left semi-ferrules.

4. An apparatus as claimed in claim 1 or 2, **characterized in that** a first scalariform detent block (41) is installed at a tip of the opening with the conjoined upper blade part, while a second scalariform detent block (42) is installed at a tip of the opening with the conjoined lower blade part, and said first scalariform detent block interacts with the second scalariform detent block, whereby said first scalariform detent block lies under the conjoined upper blade part (3111 A), while the second scalariform detent block lies above the conjoined lower blade part (3111B).

5. An apparatus as claimed in claim 1 or 2, **characterized in that** the said fixture device (3) is composed of two semi-ferrules (3A, 3B) and a connecting buckle (7), said two semi-ferrules are inter-connected at one end through the connecting buckle, while the other ends of the two semi-ferrules are locked with a screw rod (8), whereby one of the semi-ferrules has the said conjoined upper blade part (3111A) and the other semi-ferrule has the said conjoined lower blade part (3111 B) that corresponds with the conjoined upper blade part wherein round angles (311) are provided at the edges of the conjoined upper blade part and the conjoined lower blade part.

6. An apparatus as claimed in claim 1 or 2, **characterized in that** the said fixture device is composed of two semi-ferrules (3A, 3B) and a connecting buckle (7), said two semi-ferrules are inter-connected at one end through the connecting buckle, while the other ends of the two semi-ferrules are provided with L-shaped parts (411, 412) interacting with each other, and a connecting rod shaft (413) is installed at an upper one of the L-shaped parts, and an attachment hole (414) is provided at a lower one of the L-shaped parts, whereby said connecting rod shaft interacts with the attachment hole of the other semi-ferrule, said L-shaped part further comprising a toothlike protuberance (415) or toothlike groove (416) provided at an inner side of an upstanding end of a said L-shaped part, where the toothlike protuberance or toothlike groove interacts with the other semi-ferrule; and one of the semi-ferrule has the said conjoined upper blade part (3111 A) and the other semi-ferrule has the said conjoined lower blade part (3111 B) that corresponds with the conjoined upper blade part, wherein round angles (311) are provided at edges of the conjoined upper blade part and the conjoined lower blade part.

7. An apparatus as claimed in claim 6, **characterized in that** at least one connecting rod shaft (413) is installed at the said L-shaped parts with flexible connection or permanent connection.

8. An apparatus as claimed in claim 1 or 2, **characterized in that** the said fixture device (3) is composed of two semi-ferrules (3A, 3B) and a connecting buckle (7) whereby these two semi-ferrules are inter-connected at one end through the connecting buckle, while the other ends of the two semi-ferrules are provided with uncinate heave (411) or slotted hole (422) respectively which interacts with the other semi-ferrule, said uncinate heave consists of a joint plate (4211) and a clamping piece (4212) which is of Λ-shaped structure wherein said joint plate connects with a tip of a said semi-ferrule (3A) at one end and with a central part of the clamping piece (4212) at the other end; and one of the semi-ferrules has the said conjoined upper blade part (3111A) and the other semi-ferrule has the said conjoined lower blade part (3111B) that corresponds with the conjoined upper blade part, and round angles (311) are provided at edges of the conjoined upper blade part and the conjoined lower blade part.

9. An apparatus as claimed in claim 1 or 2, **characterized in that** the said fixture device (3) is composed of two semi-ferrules (3A, 3B) and a connecting buckle (7) whereby these two semi-ferrules are inter-connected at one end through the connecting buckle, while the other ends of the two semi-ferrules are provided with saucer shape heave (432) respectively where said saucer shape heave contains a block (431) in the form of a bow tie and one of the semi-ferrules has the said conjoined upper blade part (3111A) and the other semi-ferrule has the said conjoined lower blade part (3111 B) that corresponds with the upper blade conjoined part and round angles (311) are provided at edges of the conjoined upper blade part and the conjoined lower blade part.

10. An apparatus as claimed in claim 1 or 2, **characterized in that** the said fixture device (3) is composed of two semi-ferrules (3A, 3B) and a connecting buckle (7) whereby said two semi-ferrules are inter-connected at one end through the connecting buckle, while the other ends of the two semi-ferrules are provided with a strip-shaped gear body (511) or strip-shaped sprocket hole (512) respectively which interacts with that of the other semi-ferrule; and one of the semi-ferrules has the said conjoined upper blade part (3111A) and the other semi-ferrule has the said conjoined lower blade part (3111B) that corresponds with the conjoined upper blade part, and round angles (311) are provided at edges of the conjoined upper blade part and the conjoined lower blade part.

## Patentansprüche

1. Gerät zum Beschneiden eines Penis, umfassend eine Halterung (3) sowie eine Glanszwinge (1);
bei der die besage Halterung schließbar ausgeführt ist und im nicht geschlossenen Zustand eine Öffnung definiert; **dadurch gekennzeichnet, dass** die Halterung mit miteinander verbundenen oberen und unteren Klingenteilen (311A, 311B) ausgestattet ist, wobei die oberen und unteren Klingenteile auf den beiden Seiten der besagten Öffnung angeordnet sind und die freien Enden der oberen und unteren Klingenteile gerundet (311) ausgeführt sind, um ein Einklemmen der Vorhaut während der Operation und ein Überlappen während der Benutzung zu verhindern.

2. Gerät gemäß Anspruch 1, **dadurch gekennzeichnet, dass** wenigstens zwei erste Nuten (12) an einem Rand der Glanszwinge angeordnet sind und zwischen den besagten ersten Nuten eine erste Protuberanz (13) gebildet wird;
dass wenigstens zwei mit den besagten ersten Nuten in Wechselwirkung stehende zweite Protuberanzen (32) an einem Innnenrand der Halterung (1) angeordnet sind und eine zweite Nut (33) zwischen den zweiten Protuberanzen gebildet wird;
und dass die zweiten Nuten und die ersten Protuberanzen zusammenwirken, sodass ein Hohlraum für die Aufnahme einer Medikamentlösung sowie eine Passage (34) zur Injektion und als Verbindung mit dem Hohlraum entstehen.

3. Gerät gemäß Anspruch 1 oder 2, **dadurch gekennzeichnet, dass** die Glanszwinge (1) aus rechten und linken Zwingenhälften (11, 12) zusammengesetzt ist, die voneinander getrennt angeordnet sind, und dass an der Verbindungsstelle zwischen der rechten und linken Zwingenhälfte ein Kreiswinkelstück angeordnet ist.

4. Gerät gemäß Anspruch 1 oder 2, **dadurch gekennzeichnet, dass** ein erster, leiterähnlicher Arretierungsblock (41) an einer Spitze der Öffnung mit dem verbundenen oberen Klingenteil installiert ist, während ein zweiter, leiterähnlicher Arretierungsblock (42) an einer Spitze der Öffnung mit dem verbundenen unteren Klingenteil installiert ist, und dass der besagte erste leiterähnliche Arretierungsblock mit dem zweiten leiterähnlichen Arretierungsblock zusammenwirkt, sodass der erste leiterähnliche Arretierungsblock unter dem mit ihm verbundenen Klingenteil (3111 A) zu liegen kommt, während der zweite leiterähnliche Arretierungsblock über dem verbundenen unteren Klingenteil (3111 B) zu liegen kommt.

5. Gerät gemäß Anspruch 1 oder 2, **dadurch gekennzeichnet, dass** die besagte, Haltevorrichtung (3) aus zwei Zwingenhälften (3A, 3B) und einer Verbindungsspange (7) besteht, die besagten beiden Zwingenhälften an einem Ende über die Verbindungsspange miteinander verbunden sind, während die anderen Enden der beiden Zwingenhälften mit einem Schraubstift (8) miteinander verriegelt werden, sodass eine der Zwingenhälften den besagten oberen Klingenteil (3111A) und die andere Zwingenhälfte den besagten unteren Klingenteil (311B) aufweist, der dem verbundenen oberen Klingenteil entspricht, wobei an den Rändern des verbundenen oberen Klingenteils und des verbundenen unteren Klingenteils Kreiswinkelstücke (311) angeordnet sind.

6. Gerät gemäß Anspruch 1 oder 2, **dadurch gekennzeichnet, dass** die besagte Haltevorrichtung aus zwei Zwingenhälften (3A, 3B) und einer Verbindungsspange (7) besteht, die besagten beiden Zwingenhälften an einem Ende über die Verbindungsspange (7) miteinander verbunden sind, während die anderen Enden L-förmige Teile (411, 412) aufweisen, die in Wechselwirkung stehen und eine Verbindungswelle (413) am einem oberen der L-förmigen Teile montiert ist und eine Befestigungsbohrung (414) am unteren der L-förmigen Teile montiert ist, sodass die besagte Verbindungswelle in Wechselwirkung mit der Befestigungsbohrung der anderen Zwingenhälfte steht, das besagte L-förmige Teil des Weiteren einen zahnförmigen Vorsprung (415) oder eine zahnförmige Nut (416) auf der Innenseite eines stehenden Endes des besagten L-förmigen Teils aufweist, wo der zahnförmige Vorsprung oder die zahnförmige Nut mit der andere Zwingenhälfte zusammenwirken, und wo eine der Zwingenhälften den besagten verbundenen oberen Klingenteil (3111A) und die andere Zwingenhälfte den besagten unteren Klingenteil (31111 B) aufnimmt, der dem verbundenen oberen Klingenteil entspricht, wobei an den Rändern des verbundenen oberen Klingenteils und des verbundenen unteren Klingenteils Kreiswinkelstücke (311) angeordnet sind.

7. Gerät gemäß Anspruch 6, **dadurch gekennzeichnet, dass** wenigstens eine Verbindungswelle (413) an den besagten L-förmigen Teilen mit flexibler oder permanenter Verbindung ausgeführt ist.

8. Gerät gemäß Anspruch 1 oder 2, **dadurch gekennzeichnet, dass** die besagte Haltevorrichtung (3) aus zwei Zwingenhälften (3A, 3B) und einer Verbindungsspange (7) besteht, wobei diese besagten beiden Zwingenhälften an einem Ende über die Verbindungsspange miteinander verbunden sind, während an den anderen Enden der beiden Zwingenhälften ein hakenförmiges Element (411) bzw. ein Langloch(422) für eine Wechselwirkung mit der jeweils anderen Zwinge angeordnet ist, wobei das besagte hakenförmige Element aus einer Verbindungsplatte (4211) und einem Spannelement (4212) in Λ-artiger Ausführung besteht, wobei die besagte Verbindungsplatte mit einer Spitze der besagten Zwingenhälfte (3A) am einen Ende und einem Mittelteil des Spannelements (4212) am anderen Ende verbunden wird und eine der Zwingenhälften das besagte verbundene obere Klingenteil (3111A) und die andere Zwingenhälfte das verbundenen untere Klingenteil (3111 B) umfasst, das dem verbundenen oberen Klingenteil entspricht und runde Winkelstücke (311) an Rändern des verbundenen oberen Klingenteils und des verbundenen unter Klingenteils angeordnet sind.

9. Gerät gemäß Anspruch 1 oder 2, **dadurch gekennzeichnet, dass** die besagte Haltevorrichtung (3) aus zwei Zwingenhälften (3A, 3B) und einer Verbindungsspange (7) besteht, wobei diese besagten beiden Zwingenhälften an einem Ende über die Verbindungsspange miteinander verbunden sind, während an den anderen Enden der beiden Zwingenhälften ein pfannenförmiges Schließelement (432) angeordnet ist, bzw. das besagte pfannenförmige Schließelement einen Block (431) in Form einer Fliege aufweist, und eine der Zwingenhälften das besagte verbundene obere Klingenteil (3111A) und die andere Zwingenhälfte das verbundenen untere Klingenteil (3111 B) aufweist, das dem verbundenen oberen Klingenteil entspricht und Kreiswinkelstücke (311) an Rändern des verbundenen oberen Klingenteils und des verbundenen unter Klingenteils angeordnet sind.

10. Gerät gemäß Anspruch 1 oder 2, **dadurch gekennzeichnet, dass** die besagte Haltevorrichtung (3) aus zwei Zwingenhälften (3A, 3B) und einer Verbindungsspange (7) besteht, wobei die besagten beiden Zwingenhälften an einem Ende über die Verbindungsspange miteinander verbunden sind, während die anderen Enden der beiden Zwingenhälften ein bandförmiges Zahnstangenstück (511) bzw. ein bandförmiges Zahnlochstück (512) aufweisen, das in Wechselwirkung mit dem der jeweils anderen Zwingenhälfte steht, wobei eine der besagten Zwingenhälften das verbundene obere Klingenteil (3111A) und die andere Zwingenhälfte das verbundene untere Klingenteil (3111 B) umfasst, das dem verbundenen oberen Klingenteil entspricht und runde Winkelstücke (311) an Rändern des verbundenen oberen Klingenteils und des verbundenen unter Klingenteils angeordnet sind.

## Revendications

1. Appareil de circoncision du pénis, comportant :
un dispositif de fixation (3) et une bague de gland (1) ;
dans lequel ledit dispositif de fixation est en mesure d'être fermé, et quand il n'est pas fermé il définit une ouverture ;
**caractérisé en ce que** le dispositif de fixation comporte des parties conjuguées constituées d'une lame supérieure et d'une lame inférieure (3111A, 3111B), les parties constituées d'une lame supérieure et d'une lame inférieure étant agencées de chaque côté de ladite ouverture,
et **en ce que** les extrémités libres des parties constituées d'une lame supérieure et d'une lame inférieure sont arrondies (311) pour empêcher le prépuce d'être pincé lors de l'intervention et se chevauchent lors de l'utilisation.

2. Appareil selon la revendication 1, **caractérisé en ce qu'**au moins deux premières rainures (12) sont mises en oeuvre au niveau d'un bord de la bague de gland et une première partie saillante (13) est formée entre lesdites premières rainures ;
au moins deux secondes parties saillantes (32) qui coopèrent avec lesdites premières rainures sont mises en oeuvre au niveau d'un bord intérieur du dispositif de fixation (1) et une seconde rainure (33) est formée entre les secondes parties saillantes ;
les secondes rainures et les premières parties saillantes coopèrent pour former une cavité à des fins de retenue d'une solution médicamenteuse ;
et un passage (34) à des fins d'injection est formé sur le dispositif de fixation et est en interconnexion avec la cavité.

3. Appareil selon la revendication 1 ou la revendication 2, **caractérisé en ce que** ladite bague de gland (1) est composée de demi-bagues côté droit et côté gauche (11, 12) qui sont séparées l'une par rapport à l'autre, et un angle arrondi est mis en oeuvre au niveau d'une connexion des demi-bagues côté droit et côté gauche.

4. Appareil selon la revendication 1 ou la revendication 2, **caractérisé en ce qu'**un premier bloc cranté scalariforme (41) est installé au niveau d'un bout de l'ouverture comportant la partie de lame supérieure conjuguée, alors qu'un second bloc cranté scalariforme (42) est installé au niveau d'un bout de l'ouverture comportant la partie de lame inférieure conjuguée, et ledit premier bloc cranté scalariforme coopère avec le second bloc cranté scalariforme, ce par quoi ledit premier bloc cranté scalariforme repose en dessous de la partie de lame supérieure conjuguée (3111A), alors que le second bloc cranté scalariforme repose au-dessus de la partie de lame inférieure conjuguée (3111B).

5. Appareil selon la revendication 1 ou la revendication 2, **caractérisé en ce que** ledit dispositif de fixation (3) est composé de deux demi-bagues (3A, 3B) et d'une boucle de connexion (7), lesdites deux demi-bagues sont interconnectées au niveau d'une extrémité au travers de la boucle de connexion, alors que les autres extrémités des deux demi-bagues sont verrouillées au moyen d'une tige filetée (8), ce par quoi l'une des demi-bagues a ladite partie de lame supérieure conjuguée (3111A) et l'autre demi-bague a ladite partie de lame inférieure conjuguée (3111B) qui correspond à la partie de lame supérieure conjuguée dans lequel des angles arrondis (311) sont mis en oeuvre au niveau des bords de la partie de lame supérieure conjuguée et de la partie de lame inférieure conjuguée.

6. Appareil selon la revendication 1 ou la revendication 2, **caractérisé en ce que** ledit dispositif de fixation est composé de deux demi-bagues (3A, 3B) et d'une boucle de connexion (7), lesdites deux demi-bagues sont interconnectées au niveau d'une extrémité au travers de la boucle de connexion, alors que les autres extrémités des deux demi-bagues comportent des parties en forme de L (411, 412) coopérant l'une avec l'autre, et un arbre de tige de connexion (413) est installé au niveau d'une partie supérieure parmi les parties en forme de L, et un trou d'attache (414) est mis en oeuvre au niveau d'une partie supérieure parmi les parties en forme de L, ce par quoi ledit arbre de tige de connexion coopère avec le trou d'attache de l'autre demi-bague, ladite partie en forme de L comportant par ailleurs une partie saillante similaire à une dent (415), ou rainure similaire à une dent (416), mise en oeuvre au niveau d'un côté intérieur d'une extrémité verticale d'une dite partie en forme de L, où la partie saillante similaire à une dent, ou rainure similaire à une dent, coopère avec l'autre demi-bague ; et l'une des demi-bagues a ladite partie de lame supérieure conjuguée (3111A) et l'autre demi-bague a ladite partie de lame inférieure conjuguée (3111B) qui correspond à la partie de lame supérieure conjuguée, dans lequel des angles arrondis (311) sont mis en oeuvre au niveau de bords de la partie de lame supérieure conjuguée et de la partie de lame inférieure conjuguée.

7. Appareil selon la revendication 6, **caractérisé en ce qu'**au moins un arbre de tige de connexion (413) est installé au niveau desdites parties en forme de L au moyen d'une connexion flexible ou d'une connexion permanente.

8. Appareil selon la revendication 1 ou la revendication 2, **caractérisé en ce que** ledit dispositif de fixation (3) est composé de deux demi-bagues (3A, 3B) et d'une boucle de connexion (7) ce par quoi ces deux demi-bagues sont interconnectées au niveau d'une extrémité au travers de la boucle de connexion, alors que les autres extrémités des deux demi-bagues comportent une boursouflure unciforme (411) ou un trou allongé (422) respectivement coopérant avec l'autre demi-bague, ladite boursouflure unciforme étant constituée d'une plaque de jonction (4211) et d'une pièce de serrage (4212) qui est une structure en forme de , dans lequel ladite plaque de jonction se connecte avec un bout d'une dite demi-bague (3A) au niveau d'une extrémité et avec une partie centrale de la pièce de serrage (4212) au niveau de l'autre extrémité ; et l'une des demi-bagues a ladite partie de lame supérieure conjuguée (3111A) et l'autre demi-bague a ladite partie de lame inférieure conjuguée (3111B) qui correspond à la partie de lame supérieure conjuguée, et des angles arrondis (311) sont mis en oeuvre au niveau de bords de la partie de lame supérieure conjuguée et de la partie de lame inférieure conjuguée.

9. Appareil selon la revendication 1 ou la revendication 2, **caractérisé en ce que** ledit dispositif de fixation (3) est composé de deux demi-bagues (3A, 3B) et d'une boucle de connexion (7) ce par quoi ces deux demi-bagues sont interconnectées au niveau d'une extrémité au travers de la boucle de connexion, alors que les autres extrémités des deux demi-bagues comportent une boursouflure en forme de soucoupe (432) respectivement où ladite boursouflure en forme de soucoupe contient un bloc (431) sous la forme d'un oeud papillon et l'une des demi-bagues a ladite partie de lame supérieure conjuguée (3111A) et l'autre demi-bague a ladite partie de lame inférieure conjuguée (3111B) qui correspond à la partie de lame supérieure conjuguée, et des angles arrondis (311) sont mis en oeuvre au niveau de bords de la partie de lame supérieure conjuguée et de la partie de lame inférieure conjuguée.

10. Appareil selon la revendication 1 ou la revendication 2, **caractérisé en ce que** ledit dispositif de fixation (3) est composé de deux demi-bagues (3A, 3B) et d'une boucle de connexion (7) ce par quoi lesdites deux demi-bagues sont interconnectées au niveau d'une extrémité au travers de la boucle de connexion, alors que les autres extrémités des deux demi-bagues comportent un corps d'engrenage en forme de bande (511) ou un trou de roue dentée en forme de bande (512) respectivement coopérant avec celui de l'autre demi-bague ; et l'une des demi-bagues a ladite partie de lame supérieure conjuguée (3111A) et l'autre demi-bague a ladite partie de lame inférieure conjuguée (3111B) qui correspond à la partie de lame supérieure conjuguée, et des angles arrondis (311) sont mis en oeuvre au niveau de bords de la partie de lame supérieure conjuguée et de la partie de lame inférieure conjuguée.
